(19) Europäisches Patentamt
European Patent Office
Office européen des brevets

(11) Numéro de publication: **0 005 689**
**B1**

(12) # FASCICULE DE BREVET EUROPEEN

(45) Date de publication du fascicule de brevet: **18.03.81**

(21) Numéro de dépôt: **79870012.6**

(22) Date de dépôt: **02.05.79**

(51) Int. Cl.³: **C 07 D 227/087,** C 07 D 207/26, C 07 D 207/27, C 07 D 211/76, C 07 D 223/10, C 07 D 401/06, C 07 D 403/06, C 07 D 413/06 // A61K31/395 ,(C07D227/087, 295/00)

(54) **Nouveaux acides lactame-N-acétiques et leurs amides, leurs procédés de préparation et compositions thérapeutiques.**

(30) Priorité: **08.05.78 GB 1816078**

(43) Date de publication de la demande: **28.11.79 Bulletin 79/24**

(45) Mention de la délivrance du brevet: **18.03.81 Bulletin 81/11**

(84) Etats contractants désignés: **AT BE CH DE FR GB IT LU NL SE**

(56) Documents cités:
**FR-A-2 081 508**
**FR-A-2 368 474**
**DE-B-1 081 896**

(73) Titulaire: **U C B, S.A., 4, Chaussée de Charleroi, B-1060 Bruxelles (BE)**

(72) Inventeur: **Rodriguez, Ludovic, 12, Clos Dandoy, B-1180 Bruxelles (BE)**
Inventeur: **Marchal, Lucien, 44, Rue du Cours d'Eau, B-1428 Lillois (BE)**

(74) Mandataire: **Vanderborght, Henri et al, U C B, S.A. Département D.T.B. 33, rue d'Anderlecht, B-1620 Drogenbos (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

Nouveaux acides lactame-N-acétiques et leurs amides, leurs procédés de préparation et compositions thérapeutiques

La présente invention se rapporte à des acides alpha-hydroxy- ou alpha-(hydroxyalkyl)-lactame-N-acétiques, à leurs sels pharmaceutiquement accetables et à leurs amides ainsi qu'à des procédés pour les préparer. Elle se rapporte également aux compositions thérapeutiques renfermant lesdits composés.

Selon le brevet britannique n° 1.039.113, on connaît déjà des amides d'acides lactame-N-acétiques dont le plus représentatif quant à ses propriétés thérapeutiques est le piracétam ou 2-oxo-1-pyrrolidine-acétamide, désigné ci-après par produit A.

Selon le brevet britannique n° 1.309.692, on connaît également des amides d'acides alpha-alkyl-lactame-N-acétique; un représentant typique de ces derniers composés est l'alpha-éthyl-2-oxo-1-pyrrolidineacétamide, désigné ci-après par produit B.

Ces composés possèdent des propriétés thérapeutiques intéressantes notamment à cause de leur activité sur le système nerveux central et plus particulièrement, sur les processus mnésiques.

Enfin, on connaît également des acides lactame-N-acétiques, en particulier l'acide 2-oxo-1-pyrrolidineacétique (Ber. 40 [1907], 2840-41), désigné ci-après par produit C. Mais, à la connaissance de la demanderesse, la littérature n'enseigne pas que ces acides sont doués de propriétés thérapeutiques.

On a trouvé maintenant que, lorsqu'on introduit un groupe hydroxyle en position alpha dans les acides lactame-N-acétiques ou leurs amides ou sur la chaîne alkylée latérale des acides alpha-alkyl-lactame-N-acétiques ou de leurs amides, on obtient de nouveaux composés qui possèdent les propriétés thérapeutiques avantageuses des produits décrits dans les brevets précités, mais à des doses actives beaucoup plus faibles. Ainsi, ces nouveaux composés possèdent une action sur les processus mnésiques qui est supérieure à celle des amides des acides lactame-N-acétiques correspondants, en particulier à celle des produits A et B précités. On a découvert en outre que ces nouveaux composés possèdent une activité cardiaque non négligeable.

Les nouveaux composés de la présente invention répondent à la formule générale:

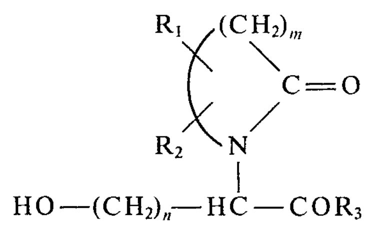

(I)

dans laquelle

$R_1$ et $R_2$ représentent indépendamment l'un de l'autre de l'hydrogène, un radical alkyle linéaire ou ramifié contenant 1 à 4 atomes de carbone, aryle non substitué ou aryle substitué par un atome d'halogène,

$R_3$ est un groupe hydroxyle ou un groupe $-NR_4R_5$ dans lequel $R_4$ et $R_5$ pris isolément représentent de l'hydrogène, un radical alkyle linéaire ou ramifié contenant 1 à 4 atomes de carbone, un radical cycloalkyle ou aralkyle, ou $R_4$ et $R_5$ pris ensemble avec l'atome d'azote auquel ils sont attachés représentent un radical hétérocyclique contenant au plus 7 chaînons, choisi parmi un radical alkylèneimino, oxaalkylèneimino, azaalkylèneimino et N-benzyl-azaalkylèneimino,

m est égal à 3, 4 ou 5, de préférence 3 et

n est égal à 0, 1 ou 2, de préférence 2.

Lorsque m est égal à 3, 4 ou 5, les composés de formule I sont respectivement des dérivés de la 2-pyrrolidinone, de la 2-pipéridinone ou de la hexahydro-2H-azépin-2-one.

Comme exemples préférés de radical alkyle, on peut citer les radicaux méthyle, éthyle, propyle, isopropyle et butyle.

Comme exemple préféré de radical aryle, on peut citer le radical phényle.

Comme exemples préférés de radical cycloalkyle, on peut citer les radicaux cyclopentyle et cyclohexyle.

Comme exemple préféré de radical aralkyle, on peut citer le radical benzyle.

Le radical alkylèneimino est de préférence le radical pipéridino, le radical oxaalkylèneimino est de préférence le radical morpholino et le radical azaalkylèneimino est de préférence le radical pipérazino.

Les composés préférés conformes à l'invention sont:

- l'acide alpha-hydroxy-2-oxo-1-pyrrolidineacétique;
- l'alpha-(2-hydroxyéthyl)-2-oxo-1-pyrrolidineacétamide;
- la 4-[4-hydroxy-2-(2-oxo-1-pyrrolidinyl)butyryl]-morpholine;
- le N,N-diéthyl-alpha-(2-hydroxyéthyl)-2-oxo-1-pyrrolidineacétamide;

— le N-cyclopentyl-alpha-(2-hydroxyéthyl)-2-oxo-1-pyrrolidineacétamide;
— le N-benzyl-alpha-(2-hydroxyéthyl)-2-oxo-1-pyrrolidineacétamide.

Les composés de formule I possèdent des propriétés pharmaceutiques intéressantes. Entre autres, ils manifestent une activité bénéfique sur les processus mnésiques et une activité de protection envers les agressions du type hypoxique. Ils trouvent ainsi une première application en géropsychiatrie, domaine dans lequel interviennent essentiellement des troubles de la mémoire liés aussi bien à des altérations cellulaires dues à l'âge qu'à une diminution de l'apport d'oxygène au cerveau suite à des accidents vasculaires uniques ou répétés. Les composés de formule I trouvent également une application intéressante dans de nombreux autres domaines, comme par exemple la prévention ou le traitement des accidents cérébrovasculaires et des déficiences cardiovasculaires, les comas post-traumatiques ou toxiques, les troubles de la mémoire, les difficultés de concentration mentale, etc.

Les composés de formule I, dans laquelle n est égal à 1 ou 2 et $R_3$ représente un groupe hydroxyle, sont préparés en faisant réagir, dans un solvant inerte, un dérivé de métal alcalin d'un lactame de formule

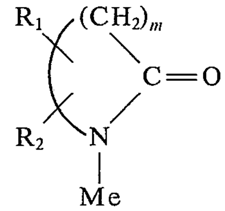

(II)

dans laquelle $R_1$, $R_2$ et m ont la signification donnée plus haut et Me représente un métal alcalin, avec une alpha-bromolactone de formule

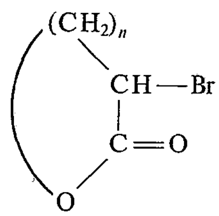

(III)

dans laquelle n a la signification donnée ci-dessus, en soumettant ensuite la lactone de l'acide alpha-(hydroxyalkyl)-lactame-N-acétique ainsi obtenue de formule

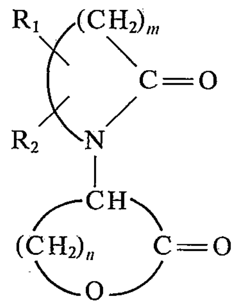

(IV)

dans laquelle $R_1$, $R_2$, m et n ont la signification donnée ci-dessus, à une hydrolyse avec un hydroxyde de métal alcalin, et en libérant finalement l'acide libre par acidification du sel de métal alcalin de l'acide alpha-(hydroxyalkyl)-lactame-N-acétique ainsi obtenu de formule

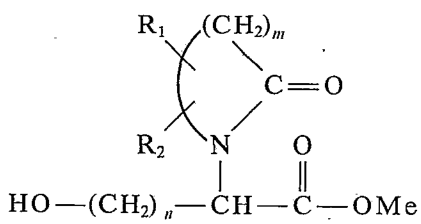

(V)

dans laquelle $R_1$, $R_2$, m et n ont la signification donnée ci-dessus et Me représente un métal alcalin.

Pour préparer les composés de formule I, dans laquelle n est égal à 1 ou 2 et $R_3$ représente un groupe $-NR_4R_5$, on synthétise d'abord de la manière décrite plus haut une lactone d'un acide

alpha-(hydroxyalkyl)-lactame-N-acétique de formule IV et on fait réagir ensuite ladite lactone avec un composé azoté de formule

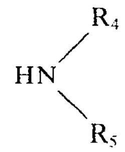

(VI)

dans laquelle $R_4$ et $R_5$ ont la même signification que plus haut.

Dans le cas particulier de la préparation de composés de formule I dans laquelle n est égal à 0 et $R_3$ représente un groupe hydroxyle, on fait réagir l'acide glyoxylique avec un lactame de formule

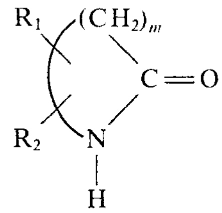

(VII)

dans laquelle $R_1$, $R_2$ et m ont la signification donnée plus haut.

Enfin, pour préparer les composés de formule I dans laquelle n est égal à 0 et $R_3$ représente un groupe $-NR_4R_5$, on fait réagir d'abord l'acide glyoxylique ou un ester de cet acide avec un lactame de formule VII et on condense ensuite l'acide alpha-hydroxy-lactame-N-acétique ainsi obtenu de formule

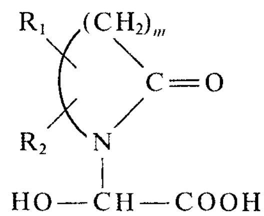

(VIII)

dans laquelle $R_1$, $R_2$ et m ont la signification donnée plus haut, ou l'ester correspondant, avec un composé azoté de formule

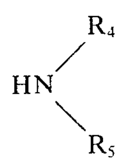

(VI)

dans laquelle $R_4$ et $R_5$ ont la signification donnée plus haut. Toutefois, lorsqu'on condense un composé azoté de formule VI avec un acide alpha-hydroxy-lactame-N-acétique de formule VIII, on doit activer au préalable ledit acide, de manière connue en soi, au moyen d'un réactif conventionnel tel que par exemple le dicyclohexylcarbodiimide.

La présente invention concerne également les sels pharmaceutiquement acceptables des acides lactame-N-acétiques de formule I. Comme exemples de tels sels, on peut citer les sels de métaux, les sels d'ammonium, les sels de bases organiques (p.ex. des sels d'amines tels que la dicyclohexylamine) et les sels d'acides aminés.

Ces sels sont obtenus selon méthodes couramment utilisées pour la préparation de ce genre de composés.

Les exemples qui suivent illustrent l'invention sans la limiter.

Dans ces exemples, la position des pics en spectroscopie infrarouge est donnée en $cm^{-1}$; en spectroscopie RMN, les déplacements chimiques sont indiqués en $\delta$ (ppm) par rapport au tétraméthylsilane, à 60 MHz.

### Exemple 1

### Préparation des lactones intermédiaires de formule IV

### 1.1. 1-(Tétrahydro-2-oxo-3-furyl)-2-pyrrolidinone

Dans 575 ml de benzène anhydre, on met en suspension 60,5 g (1,265 mole) d'hydrure de sodium (suspension commerciale à 50% dans de la paraffine préalablement lavée deux fois au benzène). A la

suspension, on ajoute goutte à goutte 98 g (1,15 mole) de 2-pyrrolidinone. On chauffe ensuite à reflux jusqu'à cessation du dégagement gazeux.

Puis on ajoute, goutte à goutte, une solution de 237 g (1,44 mole) de 3-bromodihydro-2(3H)-furanone dans 60 ml de benzène anhydre, tout en maintenant la température entre 40 et 50°C. Après que l'addition est terminée, on chauffe encore pendant 1 heure à reflux. On refroidit ensuite et on élimine le bromure de sodium par filtration. On évapore la solution benzénique sous pression réduite et l'on distille le résidu à 162—164°C/0,001 mbar. Le distillat se présente sous la forme d'un sirop qui cristallise rapidement, ce qui donne 100,8 g de 1-(tétrahydro-2-oxo-3-furyl)-2-pyrrolidinone.

P.F.: 80—81°C.
Rendement: 52%.

Analyse: $C_8H_{11}NO_3$ (P.M. = 169) en %
calculé: C 56,80 H 6,51 N 8,28
trouvé: C 56,70 H 6,58 N 8,25

Spectre infrarouge (KBr): 1785, 1770 (CO 2-oxo-furyl)
1690 (CO pyrrolidinone)

### 1.2. 3-Méthyl-1-(tétrahydro-2-oxo-3-furyl)-2-pyrrolidinone

Même préparation qu'en 1.1., sauf que l'on part de 3-méthyl-2-pyrrolidinone et que le mélange réactionnel est chauffé à reflux pendant 16 heures. Le produit obtenu distille à 140—150°C/0,006 mbar. Rendement: 8,2 g, soit 22% de la théorie; ce produit se présente sous la forme d'un sirop.

Spectre infrarouge (film): 1775 (CO 2-oxo-furyl)
1680 (CO pyrrolidinone)

### 1.3. 3-n-Butyl-1-(tétrahydro-2-oxo-3-furyl)-2-pyrrolidinone

Même préparation qu'en 1.1., sauf que l'on part de 3-n-butyl-2-pyrrolidinone et que le résidu obtenu après évaporation du solvant est utilisé sans purification pour préparer le produit de l'exemple 4.4. Rendement: 71% (produit brut).

De la même manière ont été préparées:

### 1.4. 4-p-Chlorophényl-3-phényl-1-(tétrahydro-2-oxo-3-furyl)-2-pyrrolidinone

Rendement: presque 100%

Spectre infrarouge (film): 1775 (CO 2-oxo-furyl)
1680 (CO pyrrolidinone)
825 (phényle para-substitué)
700, 750 (phényle)

### 1.5. 3,5-Diméthyl-1-(tétrahydro-2-oxo-3-furyl)-2-pyrrolidinone

Rendement: environ 10%

Spectre infrarouge (film): 1770 (CO 2-oxo-furyl)
1670 (CO pyrrolidinone)

### 1.6. 1-(Tétrahydro-2-oxo-3-furyl)-2-pipéridinone

Dans 50 ml de diméthylformamide (DMF) anhydre, on met en suspension 1,25 g (0,05 mole) d'hydrure de sodium (2,5 g d'une suspension commerciale à 50% dans de la paraffine préalablement lavée deux fois au benzène). On ajoute goutte à goutte 4,95 g (0,05 mole) de 2-pipéridinone dissous dans 20 ml de diméthylformamide. On chauffe ensuite à 60°C jusqu'à cessation du dégagement gazeux.

On refroidit et on ajoute goutte à goutte, en maintenant la température entre 5 et 10°C, 8,25 g (0,05 mole) de 3-bromodihydro-2-(3H)-furanone dissous dans 20 ml de diméthylformamide anhydre. On

agite ensuite à 60° C pendant 5 heures. Le diméthylformamide est évaporé sous pression réduite. Le résidu est repris par du chloroforme et on filtre l'insoluble sur norite. Le filtrat est à nouveau évaporé. On obtient 9,8 g d'un sirop épais.

Spectre de masse: $M^{+\cdot}$ à 183 m/e.

Le produit est employé sous cette forme dans l'exemple 4.6 pour la préparation de l'alpha-(2-hydroxyéthyl)-2-oxo-1-pipéridineacétamide.

Exemple 2
Préparation d'un acide alpha-hydroxy-lactame-N-acétique de formule I (n = 0; $R_3$ = OH)
Acide alpha-hydroxy-2-oxo-1-pyrrolidineacétique (sel de dicyclohexylamine)

On ajoute 8,5 g (0,1 mole) de 2-pyrrolidinone à 9,2 g (0,1 mole) d'acide glyoxylique monohydraté. La température s'élève spontanément jusqu'à 35° C. L'addition étant terminée, on chauffe le mélange de réaction pendant 15 minutes à 100° C. Puis on abaisse la température à 50° C et l'on ajoute 15 ml de tétrachlorure de carbone et ensuite 10 ml d'éther. Le mélange se sépare en deux phases. On sépare la phase inférieure ($CCl_4$) par décantation, on la lave deux fois avec de l'éther, puis on l'évapore sous vide. On reprend le résidu (18 g) dans de l'éthanol absolu et on ajoute à la solution 18 ml de dicyclohexylamine. Le sel de dicyclohexylamine qui précipite est recristallisé dans de l'éthanol contenant un peu d'éther. On obtient ainsi 17,7 g de sel de dicyclohexylamine de l'acide alpha-hydroxy-2-oxo-1-pyrrolidineacétique.

P.F.: 139 – 140° C. Rendement: 52%.

Analyse pour $C_{18}H_{32}N_2O_4$ (P.M. = 340) en %
calculé: C 63,58 H 9,4 N 8,24
trouvé: C 63,6 H 9,4 N 8,27

Spectra infrarouge (KBr): 3400 (OH)
1675 (CO pyrrolidinone)
1620 ($COO^-$)

Spectre de masse: $M^{+\cdot}$ (acide) inexistant, mais $M^{+\cdot}$ — COOH présent à 114 m/e.
$M^{+\cdot}$ (dicyclohexylamine) à 181 m/e.

Spectre RMN ($CDCl_3$):

| | | | |
|---|---|---|---|
| 1,0 à 3,20 | multiplet | 28 H | (deux cyclohexyle + 6 H pyrrolidinone) |
| 5,54 | singulet | 1 H | $H_{alpha}$ |
| 8 à 9 | large | 3 H | OH/COOH/NH |

Exemple 3
Préparation d'un acide alpha-(hydroxyalkyl)-lactame-N-acétique de formule I (n = 2; $R_3$ = OH)
Acide alpha-(2-hydroxyéthyl)-2-oxo-1-pyrrolidineacétique

On chauffe à reflux pendant 2 heures 67,6 g (0,4 mole) de 1-(tétrahydro-2-oxo-3-furyl)-2-pyrrolidinone et 32 g (0,8 mole) d'hydroxyde de sodium dans 210 ml d'eau, on refroidit puis on acidifie avec de l'acide chlorhydrique jusqu'à une valeur de pH de 1.

On évapore à sec le mélange sous pression réduite, on reprend le résidu trois fois dans du benzène et on évapore sous vide. Le résidu obtenu est finalement traité avec un mélange chloroforme-éthanol (4/1) et l'on filtre l'insoluble. On évapore le filtrat et l'on recristallise le résidu dans de l'éthanol. On obtient ainsi 22,7 g (rendement 30%) de l'acide alpha-(2-hydroxyéthyl)-2-oxo-1-pyrrolidineacétique.

P.F.: 123 – 124° C.

Analyse pour $C_8H_{13}NO_4$ (P.M. = 187) en %
calculé: C 51,38 H 6,95 N 7,48
trouvé: C 51,30 H 6,90 N 7,39

Spectre RMN (DMSO):

| | | | |
|---|---|---|---|
| 2,15 | multiplet | 6 H | 4 $H^{3+4}$ pyrrolidinone + 2 $H^1$ éthyle |
| 3,37 | multiplet | 4 H | 2 $H^5$ pyrrolidinone + 2 $H^2$ éthyle |
| 4,62 | quadruplet | 1 H | $H_{alpha}$ |
| 8,50 | large | 2 H | OH et COOH |

Exemples 4 à 6

Préparation des amides d'acides lactame-N-acétiques de formule I ($n = 2$; $R_3 = -NR_4R_5$)

4.1. alpha-(2-Hydroxyéthyl)-2-oxo-1-pyrrolidineacétamide

On dissout 10,15 g (0,06 mole) de 1-(tétrahydro-2-oxo-3-furyl)-2-pyrrolidinone dans 100 ml de méthanol et l'on sature la solution avec du $NH_3$. La température monte d'elle-même à 40°C. On maintient le mélange à cette température pendant 30 minutes, puis on le laisse revenir à la température ambiante. On évapore ensuite le mélange de réaction sous pression réduite et l'on recristallise la poudre obtenue dans de l'éthanol absolu. On obtient ainsi 10 g de l'alpha-(2-hydroxyéthyl)-2-oxo-1-pyrrolidineacétamide fondant à 164–165° C (rendement 90%).

Analyse pour $C_8H_{14}N_2O_3$ (P.M. = 186) en %
calculé: C 51,65 H 7,58 N 15,05
trouvé: C 51,70 H 7,60 N 14,86

Spectre infrarouge (KBr): 3340, 3180 ($NH_2$)
1695 (CO pyrrolidinone)
1650 (CO amide)
1075 (OH)

Spectre RMN (DMSO):

| | | | |
|---|---|---|---|
| 2,15 | multiplet | 6 H | 4 $H^{3+4}$ pyrrolidinone + 2 $H^1$ éthyle |
| 3,40 | multiplet | 4 H | 2 $H^5$ pyrrolidinone + 2 $H^2$ éthyle |
| 4,46 | multiplet | 2 H | OH + $H_{alpha}$ |
| 7,08 à 7,30 | large | 2 H | $CONH_2$ |

On a obtenu par la même méthode les composés 4.2 à 4.6 suivants:

4.2. alpha-(2-Hydroxyéthyl)-3-méthyl-2-oxo-1-pyrrolidineacétamide

P.F.: 101–102° C. Rendement: 86%.

Analyse pour $C_9H_{16}N_2O_3$ (P.M. = 200) en %
calculé: C 54 H 8 N 14
trouvé: C 53,71 H 7,95 N 13,92

Spectre infrarouge (KBr): 3470 (OH)
3310, 3160 ($NH_2$)
1695 (CO pyrrolidinone)
1640 ($CONH_2$)
1055 (OH)

Spectre RMN (DMSO):

| | | | |
|---|---|---|---|
| 1,05 | doublet | 3 H | $CH_3$ |
| 1,25–2,35 | multiplet | 5 H | 3 $H^{3+4}$ pyrrolidinone + 2 $H^1$ éthyle |
| 3,35 | multiplet | 4 H | 2 $H^5$ pyrrolidinone + 2 $H^2$ éthyle |
| 4,45 | multiplet | 2 H | OH + $H_{alpha}$ |
| 7,15 | doublet | 2 H | $CONH_2$ |

Spectre de masse: $M^{+\cdot}$ à 200 m/e.

4.3. alpha-(2-Hydroxyéthyl)-3,5-dimethyl-2-oxo-1-pyrrolidineacétamide

Sirop. Rendement: 51%.

Analyse pour $C_{10}H_{18}N_2O_3$ (P.M. = 214) en %
calculé: C 56,07 H 8,41 N 13,08
trouvé: C 55,5 H 8,5 N 12,92

Spectre infrarouge ($CHCl_3$): 3470 (OH)
3360, 3180 ($NH_2$)
1660 à 1690 (CO)
1050 (OH)

Spectre RMN ($CDCl_3$):

| | | | |
|---|---|---|---|
| 1,21 | multiplet | 6 H | 2 $CH_3$ |
| 1,6 à 2,9 | multiplet | 5 H | 3 $H^{3+4}$ pyrrolidinone + 2 $H^1$ éthyle |
| 3,6 | multiplet | 4 H | OH + $H^5$ pyrrolidinone + 2 $H^2$ éthyle |
| 4,30 | quadruplet | 1 H | $H_{alpha}$ |
| 6,20 à 7,30 | large | 2 H | $CONH_2$ |

Spectre de masse: $M^{+\cdot}$ à 214 m/e.

### 4.4 3-n-Butyl-alpha-(2-hydroxyéthyl)-2-oxo-1-pyrrolidineacétamide

P.F.: 90 – 91° C. Rendement: 26%.

Analyse pour $C_{12}H_{22}N_2O_3$ (P.M. = 242) en %

| | | | |
|---|---|---|---|
| calculé: | C 59,5 | H 9,09 | N 11,57 |
| trouvé: | C 59,67 | H 9,20 | N 11,54 |

Spectre infrarouge (KBr): 3390, 3340, 3180 (OH, $NH_2$)
1710 (CO pyrrolidinone)
1660 ($CONH_2$)
1050 (OH)

Spectre RMN ($CDCl_3$):

| | | | |
|---|---|---|---|
| 0,7 à 2,5 | multiplet | 13 H | $C_4H_9$ + 2 $H^4$ pyrrolidinone + 2 $H^1$ éthyle |
| 3,2 à 4,0 | multiplet | 6 H | 3 $H^{3+5}$ pyrrolidinone + 2 $H^2$ éthyle + OH |
| 4,86 | triplet | 1 H | $H_{alpha}$ |
| 6,20 et 7,05 | doublet | 2 H | $CONH_2$ |

Spectre de masse: $M^{+\cdot}$ à 242 m/e.

### 4.5. 4-p-Chlorophényl-alpha-(2-hydroxyéthyl)-3-phényl-2-oxo-1-pyrrolidineacétamide

P.F.: 60 – 61° C. Rendement: 23%.

Analyse pour $C_{20}H_{21}ClN_2O_3$ (P.M. = 372,5) en %

| | | | |
|---|---|---|---|
| calculé: | C 64,43 | H 5,64 | N 7,52 |
| trouvé: | C 63,39 | H 5,50 | N 7,64 |

Spectre infrarouge (KBr): 3360, 3200 (OH, $NH_2$)
1670 (large CO)
1050 (OH)
820 (p-chlorophényle)
700, 750 (phényle)

Spectre RMN ($CDCl_3$):

| | | | |
|---|---|---|---|
| 2,0 | large | 2 H | 2 $H^1$ éthyle |
| 3,30 à 4,10 | multiplet | 6 H | 4 $H^{3+4+5}$ pyrrolidinone + 2 $H^2$ éthyle |
| 5,0 | triplet | 1 H | $H_{alpha}$ |
| 5,85 | large | 1 H | OH |
| 7,15 | multiplet | 11 H | 9 H deux phényles + $CONH_2$ |

Spectre de masse: $M^{+\cdot}$ à 372 m/e.

### 4.6. alpha-(2-Hydroxyéthyl)-2-oxo-1-pipéridineacétamide

Les résines obtenues après évaporation du mélange réactionnel sont chromatographiées sur colonne de silice (Eluant: mélange 95 : 5 chloroforme-méthanol). On isole ainsi une poudre légèrement colorée.

Spectre infrarouge (KBr): 3430 (OH)
3180 à 3270 ($NH_2$)
1695 (CO pipéridinone)
1615 ($CONH_2$)

Spectre RMN ($CDCl_3$):

| | | | |
|---|---|---|---|
| 1,5 à 2,7 | multiplet | 8 H | 6 $H^{3+4+5}$ pipéridinone + 2 $H^1$ éthyle |
| 2,9 à 4,0 | multiplet | 5 H | 2 $H^6$ pipéridinone + 2 $H^2$ éthyle + OH |
| 5,3 | multiplet | 1 H | $H_{alpha}$ |
| 6,3 et 6,92 | doublet | 2 H | $CONH_2$ |

Spectre de masse: $M^{+\cdot}$ à 200 m/e.

5.1. N-n-Butyl-alpha-(2-hydroxyéthyl)-2-oxo-1-pyrrolidineacétamide

On dissout 10,14 g (0,06 mole) de 1-(tétrahydro-2-oxo-3-furyl)-2-pyrrolidinone dans 50 ml de méthanol et l'on ajoute à la solution 8,78 g (0,12 mole) de n-butylamine.

On chauffe le mélange à reflux (65° C) pendant 3 heures et l'on évapore ensuite sous vide poussé. On obtient 12,3 g (85% de rendement) de N-n-butyl-alpha-(2-hydroxyéthyl)-2-oxo-1-pyrrolidineacétamide, lequel se présente sous la forme d'un sirop.

Analyse pour $C_{12}H_{22}N_2O_3$ (P.M. = 242) en %

| | | | |
|---|---|---|---|
| calculé: | C 59,5 | H 9,09 | N 11,6 |
| trouvé: | C 59,32 | H 9,09 | N 11,54 |

Spectre infrarouge (film): 3440 (OH)
3300 (NH)
1690 à 1640 (CO)
1540 (NH)
1055 (OH)

Spectre RMN ($CDCl_3$):

| | | | |
|---|---|---|---|
| 0,8 à 2,8 | multiplet | 13 H | |
| 3,0 à 4,0 | multiplet | 7 H | 2 $H^5$ pyrrolidinone + N—$CH_2$ (butyle) + 2 $H^2$ éthyle + OH |
| 4,88 | triplet | 1 H | $H_{alpha}$ |
| 7,15 | triplet | 1 H | NH |

Spectre de masse: $M^{+\cdot}$ à 242 m/e.

Les composés 5.2 à 5.4 qui suivent ont été Préparés de manière analogue.

5.2. N-Cyclohexyl-alpha-(2-hydroxyéthyl)-2-oxo-1-pyrrolidineacétamide

P.F.: 122 – 123° C. Rendement: 73%

Analyse pour $C_{14}H_{24}N_2O_3$ (P.M. = 268) en %

| | | | |
|---|---|---|---|
| calculé: | C 62,7 | H 8,95 | N 10,44 |
| trouvé: | C 62,52 | H 8,94 | N 10,42 |

Spectre infrarouge (KBr): 3500 (OH)
3300 (NH)
1660 (CO)
1530 (NH)
1050 (OH)

Spectre RMN ($CDCl_3$):

| | | | |
|---|---|---|---|
| 0,9 à 2,8 | multiplet | 16 H | 4 $H^{3+4}$ pyrrolidinone + 2 $H^1$ éthyle + 10 H des $CH_2$ cyclohexyle |
| 3,30 à 4,0 | multiplet | 6 H | 2 $H^5$ pyrrolidinone + 2 $H^2$ éthyle + CH du cyclohexyle + OH |
| 4,85 | triplet | 1 H | $H_{alpha}$ |
| 6,98 | doublet | 1 H | NH |

Spectre de masse: $M^{+\cdot}$ à 268 m/e.

5.3. N,N-Diéthyl-alpha-(2-hydroxyéthyl)-2-oxo-1-pyrrolidineacétamide

Sirop. Rendement: 33%.

Spectre infrarouge (film): 3420 (OH)
1660 (CO pyrrolidinone)
1635 (CO amide)
1055 (OH)

Spectre RMN ($CDCl_3$):

| | | | |
|---|---|---|---|
| 1,1 à 1,18 | 2 triplets | 6 H | 2 $CH_3$ (diéthyle) |
| 1,7 à 2,6 | multiplet | 6 H | 4 $H^{3+4}$ pyrrolidinone + 2 $H^1$ éthyle |
| 3,1 à 3,8 | multiplet | 9 H | 2 $CH_2$ (diéthyle) + 2 $H^5$ pyrrolidinone + 2 $H^2$ éthyle + OH |
| 5,18 | triplet | 1 H | $H_{alpha}$ |

Spectre de masse: $M^{+\cdot}$ à 242 m/e.

### 5.4. alpha-(2-Hydroxyéthyl)-N-isopropyl-2-oxo-1-pyrrolidineacétamide

Sirop. Rendement: 83%.

Analyse pour $C_{11}H_{20}N_2O_3$ (P.M. = 228) en %

| | | | |
|---|---|---|---|
| calculé: | C 57,89 | H 8,17 | N 12,28 |
| trouvé: | C 55,08 | H 8,38 | N 12,76 |

Spectre infrarouge (film): 3420 (OH)
3300 (NH)
1660 à 1690 (CO)
1540 (NH)
1055 (OH)

Spectre RMN ($CDCl_3$):

| | | | |
|---|---|---|---|
| 1,12 | doublet | 6 H | 2 $CH_3$ (isopropyle) |
| 1,7 à 2,6 | multiplet | 6 H | 4 $H^{3+4}$ pyrrolidinone + 2 $H^1$ éthyle |
| 3,3 à 4,2 | multiplet | 6 H | 2 $H^5$ pyrrolidinone + 2 $H^2$ éthyle + CH (isopropyl) + OH |
| 4,80 | triplet | 1 H | $H_{alpha}$ |
| 7,0 | doublet | 1 H | NH |

Spectre de masse: $M^{+\cdot}$ à 228 m/e.

### 6.1. 4-[4-Hydroxy-2-(2-oxo-1-pyrrolidinyl)butyryl]-morpholine

On mélange 5,07 g (0,03 mole) de 1-(tétrahydro-2-oxo-3-furyl)-2-pyrrolidinone avec 10,45 g (0,12 mole) de morpholine et l'on chauffe le mélange à 110° C pendant 5 heures. Au refroidissement le produit cristallise.

On filtre et on recristallise celui-ci dans de l'éther. On obtient ainsi 6,3 g (rendement 82%) de 4-[4-hydroxy-2-(2-oxo-1-pyrrolidinyl)-butyryl]-morpholine. P.F.: 105—106° C.

Analyse pour $C_{12}H_{20}N_2O_4$ (P.M. = 256) en %

| | | | |
|---|---|---|---|
| calculé: | C 56,25 | H 7,8 | N 10,93 |
| trouvé: | C 56,15 | H 7,82 | N 10,90 |

Spectre infrarouge (KBr): 3450 (OH)
1680 (CO pyrrolidinone)
1650 (CO amide)
1050 (OH)

Spectre RMN ($CDCl_3$):

| | | | |
|---|---|---|---|
| 1,8 à 2,6 | multiplet | 6 H | 4 $H^{3+4}$ pyrrolidinone + 2 $H^3$ butyryle |
| 3 à 4 | multiplet | 13 H | 2 $H^5$ pyrrolidinone + 8 H morpholine + 2 $H^4$ butyryle + OH |
| 5,2 | triplet | 1 H | $H^2$ butyryle |

Spectre de masse: $M^{+\cdot}$ à 256 m/e.

Les composés 6.2 à 6.6 qui suivent ont été préparés de manière analogue.

### 6.2. 1-[4-Hydroxy-2-(2-oxo-1-pyrrolidinyl)butyryl]-pipéridine

P.F.: 129° C. Rendement: 89%.

Analyse pour $C_{13}H_{22}N_2O_3$ (P.M. = 254) en %

| | | | |
|---|---|---|---|
| calculé: | C 61,4 | H 8,66 | N 11,02 |
| trouvé: | C 61,21 | H 8,59 | N 11,0 |

Spectre infrarouge (KBr): 3420 (OH)
1680 (CO pyrrolidinone)
1625 (CO amide)
1055 (OH)

Spectre RMN ($CDCl_3$):

| | | | |
|---|---|---|---|
| 1,3 à 2,7 | multiplet | 12 H | 4 $H^{3+4}$ pyrrolidinone + 6 H pipéridine + 2 $H^3$ butyryle |
| 3,2 à 4,0 | multiplet | 9 H | 2 $H^5$ pyrrolidinone + 4 H pipéridine + 2 $H^4$ butyryle + OH |
| 5,2 | triplet | 1 H | $H^2$ butyryle |

Spectre de masse: $M^{+\cdot}$ à 254 m/e.

6.3. alpha-(2-Hydroxyéthyl)-N-propyl-2-oxo-1-pyrrolidineacétamide

Sirop. Rendement: 98%.

Analyse pour $C_{11}H_{20}N_2O_3$ (P.M. = 228) en %
calculé: C 57,89 H 8,77 N 12,28
trouvé: C 56,96 H 8,60 N 12,46

Spectre infrarouge (film): 3420 (OH)
3300 (NH)
1650 – 1690 (CO)
1535 (NH)
1055 (OH)

Spectre RMN ($CDCl_3$):

| | | | |
|---|---|---|---|
| 0,92 | triplet | 3 H | $CH_3$ (propyle) |
| 1,2 à 2,7 | multiplet | 8 H | 4 $H^{3+4}$ pyrrolidinone + $CH_2$ (propyle) + 2 $H^1$ éthyle |
| 3,0 à 4,1 | multiplet | 7 H | 2 $H^5$ pyrrolidinone + $CH_2$ (propyle) + 2 $H^2$ éthyle + OH |
| 4,9 | triplet | 1 H | $H_{alpha}$ |
| 7,20 | triplet | 1 H | NH |

Spectre de masse: $M^{+\cdot}$ à 228 m/e.

6.4. 1-Benzyl-4-[4-hydroxy-2-(2-oxo-1-pyrrolidinyl)butyryl]-pipérazine

Sirop. Rendement: 67%.

Spectre infrarouge (film): 3420 (OH)
1640 à 1690 (CO)
1055 (OH)
745, 700 (phényle)

Spectre RMN ($CDCl_3$):

| | | | |
|---|---|---|---|
| 1,6 à 3,8 | multiplet | 21 H | |
| 5,18 | triplet | 1 H | $H^2$ butyryle |
| 7,28 | singulet | 5 H | 5 H phényle |

Spectre de masse: $M^{+\cdot}$ à 345 m/e.

6.5. N-Cyclopentyl-alpha-(2-hydroxyéthyl)-2-oxo-1-pyrrolidineacétamide

Le sirop obtenu après évaporation sous pression réduite du mélange réactionnel, est purifié par chromatographie sur colonne de silice (Eluant: mélange 95 : 5 chloroforme-méthanol).

Les fractions convenables sont réunis, évaporées, et le sirop residuel est trituré dans de l'éther contenant quelques gouttes de chloroforme. Le N-cyclopentyl-alpha-(2-hydroxyéthyl)-2-oxo-1-pyrrolidineacétamide cristallise.

P.F.: 81 – 83° C. Rendement: 72%.

Analyse pour $C_{13}H_{22}N_2O_3$ (P.M. = 254) en %
calculé: C 61,41 H 8,66 N 11,02
trouvé: C 61,44 H 8,66 N 10,98

Spectre infrarouge (KBr): 3450 (OH)
3260 (NH)
1680 (CO pyrrolidinone)
1650 (CO amide)
1550 (NH amide)
1060 (OH)

Spectre RMN ($CDCl_3$):

| | | | |
|---|---|---|---|
| 1,6 à 2,8 | multiplet | 14 H | 8 H des $CH_2$ cyclopentyle + 4 $H^{3+4}$ pyrrolidinone + 2 $H^1$ éthyle |
| 3,3 à 4,4 | multiplet | 6 H | 2 $H^5$ pyrrolidinone + 2 $H^2$ éthyle + CH du cyclopentyle + OH |
| 4,85 | triplet | 1 H | $H_{alpha}$ |
| 7,20 | doublet | 1 H | NH |

Spectre de masse: $M^{+\cdot}$ à 254 m/e.

### 6.6 N-Benzyl-alpha-(2-hydroxyéthyl)-2-oxo-1-pyrrolidineacétamide

Le sirop obtenu après évaporation du mélange réactionnel cristallise très lentement. On lave les cristaux à l'éther.

P.F.: 90–92° C. Rendement: 71%.

Analyse pour $C_{15}H_{20}N_2O_3$ (P.M. = 276) en %

| | | | |
|---|---|---|---|
| calculé: | C 65,21 | H 7,24 | N 10,14 |
| trouvé: | C 65,02 | H 7,43 | N 10,23 |

Spectre infrarouge (film): 3400 (OH)
3300 (NH)
1640 à 1690 (CO)
1540 (NH)
1065 (OH)
710 (phényle)

Spectre RMN ($CDCl_3$):

| | | | |
|---|---|---|---|
| 1,7 à 2,5 | multiplet | 6 H | 4 $H^{3+4}$ pyrrolidinone + 2 $H^1$ éthyle |
| 3,2 à 3,9 | multiplet | 5 H | 2 $H^5$ pyrrolidinone + 2 $H^2$ éthyle + OH + triplet |
| 4,4 | doublet | 2 H | $CH_2$ (benzyle) |
| 4,9 | triplet | 1 H | $H_{alpha}$ |
| 7,23 | singulet | 5 H | 5 H phényle |
| 7,6 | triplet | 1 H | NH |

Spectre de masse: $M^{+\cdot}$ à 276 m/e.

## Essais pharmacologiques

Les produits de la présente invention ont été soumis à des tests pharmacologiques dont les résultats sont reproduits ci-après.

### I. Action sur les processus mnésiques

A) L'action sur les processus mnésiques est démontrée en premier lieu par l'aptitude des produits à améliorer un type de rétention mémorielle chez le rat. Le principe du test d'évitement actif (voir M. Greindl et S. Preat, Arch. Int. Pharmacodyn. Thérap. 223, [1976] [1], 168–171) mis au point dans les laboratoires de la demanderesse et utilisé à effet peut être décrit comme suit: on observe la réaction de retrait de la patte du rat soumise à une pression croissante et quantifiée. La pression pour laquelle la réaction se produit est appelée seuil de réaction. Ce dernier est exprimé par le nombre de graduations de l'échelle de l'appareil utilisé (Analgesymeter UGO BASILE-Milan) et correspond donc à la pression minimale qui, appliquée sur la patte des animaux, provoque le retrait. Elle se lit directement sur l'échelle de l'appareil utilisé.

Testés 24 heures plus tard, les animaux témoins ne montrent aucune rétention apparente de l'épreuve antérieure: l'évitement se produit pour une intensité de stimulation comparable à celle de la veille. Inversement, des animaux traités avex une substance ayant un effet positif sur les processus mnésiques (comme par exemple le piracétam) montrent un degré significatif de rétention: le stimulus auquel les rats réagissent par un réflexe d'évitement est statistiquement inférieur à celui des témoins.

On utilise un minimum de 20 rats par test (10 rats traités et 10 rats témoins) et on définit comme dose active la dose minimale abaissant le stimulus au-dessous de 11 graduations.

L'administration par voie sous-cutanée de certains composés de formule I a donné dans ces conditions les résultats repris au tableau suivant:

| Produit de l'exemple | dose active en mmol/kg |
|---|---|
| 2 | 0,005 |
| 4.1 | 0,002 |
| 5.3 | 0,0002 |
| 6.1 | 0,0002 |
| 6.3 | 0,0001 |
| 6.4 | 0,001 |
| 6.5 | 0,0002 |
| 6.6 | 0,002 |
| Produit A (comparaison) | 0,025 |
| Produit B (comparaison) | 0,005 |

Le produit C (comparaison) est inactif à une dose de 0,1 mmol/kg.

L'examen de ce tableau montre que ces composés manifestent tous dans ce test une activité supérieure à celle des produits A et B dont l'action sur les processus mnésiques est bien connue.

B) L'action sur les processus mnésiques est également démontrée par la réduction du temps de fixation spinale, test qui a été décrit dans la littérature (C. Giurgea et F. Mouravieff-Lesuisse, Arch. Int. Pharmacodyn. 191, ( [2], [1971], 279) comme un modèle élémentaire de mémoire et qui est doué d'une réactivité pharmacologique en bonne corrélation avec la physiopathologie clinique. Chez le rat, après lésion unilatérale du cervelet, il y a asymétrie posturale des pattes postérieures. Cette asymétrie peut persister même après section spinale si l'animal a passé un temps suffisamment long dans cette position. Ce temps, dit de fixation spinale, est dans les conditions expérimentales appliquées ici de 45 minutes.

Par contre, si la section spinale est pratiquée avant l'échéance de cet intervalle, par exemple 35 minutes après l'installation de l'asymétrie, cette dernière disparaît.

Aucun animal traité par des placébos ne conserve l'asymétrie dans ces conditions.

Inversement, tout produit permettant aux rats de garder l'asymétrie (donc réalisant la fixation spinale) lorsque la section spinale est effectuée après 35 minutes est considéré comme actif.

L'administration par voie intrapéritonéale de certains composés de formule I a donné dans ces conditions les résultats repris au tableau suivant. Par »nombre d'animaux« on entend le nombre d'animaux ayant répondu positivement au test par rapport au nombre d'animaux testés à la dose indiquée.

| Produit de l'exemple | Dose active mmol/kg | Nombre d'animaux |
|---|---|---|
| 2 | 0,1 | 4/7 |
| 3 | 0,1 | 4/7 |
| 4.1 | 0,1 | 3/7 |
| 6.6 | 0,1 | 3/7 |
| Produit A (comparaison) | 0,2 | 4/9 |
| Produit B (comparaison) | 0,2 | 7/22 |
| Produit C (comparaison) | 0,32 | 2/4 |

Il apparaît que les composés de l'invention ont la même activité que les produits de comparaison, mais à une dose nettement plus faible.

## II. Activité cardiaque

Il a été observé que les composés de l'invention possèdent une activité cardiaque non négligeable; celle-ci a été mise en évidence dans le test du »Muscle papillaire«.

La méthode utilisée est celle de M. K. Cattell et H. Gold (J. Pharmacol. Exptl. Therap. 62, ( [1938], 116–125). On opère sur un muscle papillaire, isolé du cœur d'un chat, et qui trempe dans une solution physiologique à laquelle on ajoute le produit à tester.

Dans ce test, le composé de l'exemple 4.1 montre une activité inotrope (stimulation du fonctionnement du muscle) supérieure à celle de la caféine.

Ainsi, à une dose de 10 µg/kg, l'augmentation de la force de contraction du muscle est respectivement de 7% pour le composé de l'invention et de 4% pour la caféine.

## III. Toxicité

Les composés de l'invention sont peu toxiques. Leur toxicité en administration par voie intrapéritonéale chez la souris, est donnée dans le tableau suivant:

| Composé de l'exemple | mg/kg*) |
|---|---|
| 2 | > 102 |
| 3 | >1222 |
| 4.1 | >1116 |
| 4.2 | >1200 |
| 4.3 | >1286 |
| 4.4 | >1452 |
| 4.5 | > 224 |
| 5.1 | >1452 |
| 5.2 | >1608 |
| 5.3 | >1452 |
| 5.4 | >1368 |
| 6.1 | >1536 |
| 6.2 | >1524 |
| 6.3 | >1368 |
| 6.4 | > 345 |
| 6.5 | > 762 |
| 6.6 | = 822 |

*) Dose qui amène la mort chez un animal sur trois lors de la réalisation du test d'Irwin (S. Irwin, Gordon Research Conference on Medicinal Chemistry, Colby Junior College, New London, 1959).

D'autre part, chez le rat, en administration intraveineuse (i.v.) ou per os, les composés de l'invention se sont révélés également peu toxiques; ainsi par exemple:

| Composé de l'exemple | Voie d'administration | DL 50 (mg/kg) |
|---|---|---|
| 4.1 | i.v. | > 4000 |
| 4.1 | per os | >10000 |

## IV. Posologie—Administration

Les compositions pharmaceutiques de l'invention utilisables pour l'administration orale peuvent être solides ou liquides, par exemple sous forme de comprimés, pilules, dragées, capsules en gélatine, solutions, sirops, etc. ... De même, les compositions utilisables pour l'administration par voie parentérale sont les formes pharmaceutiques connues pour ce genre d'administration, par exemple des solutions, suspensions ou émulsions aqueuses ou huileuses.

Pour l'administration par voie rectale, les compositions de l'invention se présentent généralement sous forme de suppositoires.

Les formes pharmaceutiques telles que solutions injectables, suspensions injectables, comprimés, gouttes, suppositoires, sont préparées selon les méthodes couramment utilisées par les pharmaciens.

On mélange les composés de l'invention avec un véhicule solide ou liquide, non toxique, pharmaceutiquement acceptable et éventuellement avec un agent dispersant, un agent désintégrant, un lubrifiant, un agent stabilisant, et. ... On peut y ajouter, le cas échéant, des préservatifs, des agents édulcorants, des agents colorants, etc. ...

De même, les véhicules pharmaceutiques solides ou liquides utilisés dans ces compositions sont bien connus de l'homme de métier. Des excipients pharmaceutiques solides pour la préparation de comprimés ou de capsules sont par exemple l'amidon, le talc, le carbonate de calcium, le lactose, le sucrose, le stéarate de magnésium, etc.

Le pourcentage de produit actif dans les compositions pharmaceutiques peut varier dans des limites très larges selon les conditions d'emploi, en particulier suivant la fréquence d'administration.

La posologie humaine a un ordre de grandeur de 2 × 250 mg/jour mais peut éventuellement varier de 10 mg à 4 g par jour.

A titre d'exemple non limitatif d'une composition contenant les composés de l'invention, on donne ci-après un exemple de formule pour comprimé:

| | |
|---|---|
| composé de l'exemple 4.1 | 400 mg |
| amidon | 61 mg |
| polyvinyl-pyrrolidone | 8 mg |
| talc | 26 mg |
| stéarate de Mg | 5 mg |

**Revendications pour les Etats contractants: BE, FR, GB, NL, DE, LU, SE, CH**

1. Acides lactame-N-acétiques et leurs amides répondant à la formule et IT générale

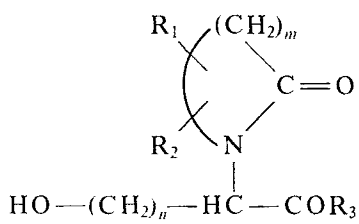

(I)

dans laquelle

$R_1$ et $R_2$ représentent indépendamment l'un de l'autre de l'hydrogène, un radical alkyle linéaire ou ramifié contenant 1 à 4 atomes de carbone, aryle non substitué ou aryle substitué par un atome d'halogène,

$R_3$ est un groupe hydroxyle ou un groupe $-NR_4R_5$ dans lequel $R_4$ et $R_5$ pris isolément représentent de l'hydrogène, un radical alkyle linéaire ou ramifié contenant 1 à 4 atomes de carbone, un radical cycloalkyle ou aralkyle, ou $R_4$ et $R_5$ pris ensemble avec l'atome d'azote auquel ils sont attachés représentent un radical hétérocyclique contenant au plus 7 chaînons, choisi parmi un radical alkylèneimino, oxaalkylèneimino, azaalkylèneimino et N-benzyl-azaalkylèneimino,

m est égal à 3, 4 ou 5,

n est égal à 0, 1 ou 2,

ainsi que les sels pharmaceutiquement acceptables desdits acides lactame-N-acétiques.

2. Acides lactame-N-acétiques, leurs sels et leurs amides selon la revendication 1, dans lesquels m est égal à 3.

3. Acide alpha-hydroxy-2-oxo-1-pyrrolidineacétique et ses sels pharmaceutiquement acceptables, selon la revendication 1.

4. alpha-(2-Hydroxyéthyl)-2-oxo-1-pyrrolidineacétamide, selon la revendication 1.

5. N,N-Diéthyl-alpha-(2-hydroxyéthyl)-2-oxo-1-pyrrolidineacétamide, selon la revendication 1.

6. 4-[4-Hydroxy-2-(2-oxo-1-pyrrolidinyl)butyryl]-morpholine, selon la revendication 1.

7. N-Cyclopentyl-alpha-(2-hydroxyéthyl)-2-oxo-1-pyrrolidineacétamide, selon la revendication 1.

8. N-Benzyl-alpha-(2-hydroxyéthyl)-2-oxo-1-pyrrolidineacétamide, selon la revendication 1.

9. Procédé de préparation d'acides lactame-N-acétiques, de leurs sels et de leurs amides tels que définis dans la revendication 1, caractérisé en ce que:

a) pour préparer les acides lactame-N-acétiques répondant à la formule générale I dans laquelle n est égal à 1 ou 2 et $R_3$ représente un groupe hydroxyle, on fait réagir, dans un solvant inerte, un

dérivé de métal alcalin d'un lactame de formule

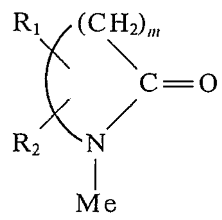

(II)

dans laquelle $R_1$, $R_2$ et m ont la signification donnée à la revendication 1 et Me représente un métal alcalin, avec une alpha-bromolactone de formule

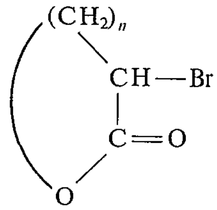

(III)

dans laquelle n à la signification donnée ci-dessus, et on soumet ensuite la lactone de l'acide alpha-(hydroxyalkyl)-lactame-N-acétique ainsi obtenue de formule

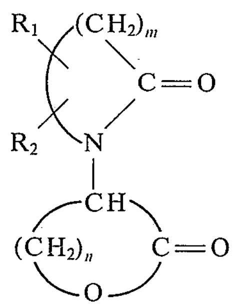

(IV)

dans laquelle $R_1$, $R_2$, m et n ont la signification donnée ci-dessus, à une hydrolyse avec un hydroxyde de métal alcalin, et on libère finalement l'acide libre par acidification du sel de métal alcalin de l'acide alpha-(hydroxyalkyl)-lactame-N-acétique ainsi obtenu de formule

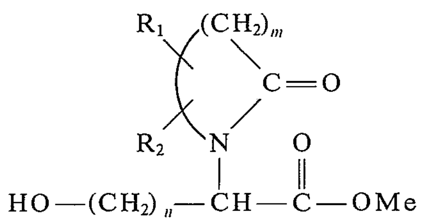

(V)

dans laquelle $R_1$, $R_2$, m et n ont la signification donnée ci-dessus et Me représente un métal alcalin; ou

b) pour préparer les amides d'acides lactame-N-acétiques répondant à la formule générale I dans laquelle n est égal à 1 ou 2 et $R_3$ représente un groupe $-NR_4R_5$, on fait réagir une lactone d'un acide alpha-(hydroxyalkyl)-lactame-N-acétique de formule IV obtenue de la manière décrite au point a) ci-dessus avec un composé azoté de formule

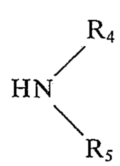

(VI)

dans laquelle $R_4$ et $R_5$ ont la signification donnée à la revendication 1; ou

c) pour préparer les acides lactame-N-acétiques répondant à la formule générale I dans laquelle n est égal à 0 et $R_3$ représente un groupe hydroxyle, on fait réagir l'acide glyoxylique avec un lactame de formule

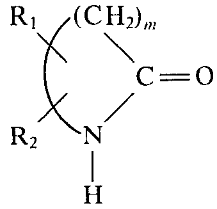

(VII)

dans laquelle $R_1$, $R_2$ et m ont la signification donnée à la revendication 1; ou

d) pour préparer les amides d'acides lactame-N-acétiques répondant à la formule générale I dans laquelle n est égal à 0 et $R_3$ représente un groupe $-NR_4R_5$, on fait réagir d'abord l'acide glyoxylique ou un ester de cet acide avec un lactame de formule VII indiquée ci-dessus et on condense ensuite l'acide alpha-hydroxylactame-N-acétique ainsi obtenu de formule

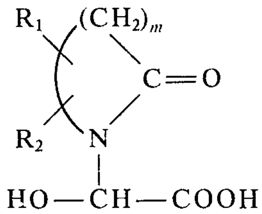

(VIII)

dans laquelle $R_1$, $R_2$ et m ont la signification donnée ci-dessus, ou l'ester correspondant, avec un composé azoté de formule VI indiquée ci-dessus; et

e) en ce que éventuellement, on convertit les acides lactame-N-acétiques de formule I obtenus aux points a) et c) ci-dessus en leurs sels pharmaceutiquement acceptables.

10. Compositions thérapeutiques renfermant une quantité thérapeutiquement efficace d'un acide lactame-N-acétique ou de son amide, ou d'un sel pharmaceutiquement acceptable dudit acide lactame-N-acétique selon la revendication 1 en association avec des excipients pharmaceutiques solides ou liquides.

**Revendication pour l'état contractant: AT**

Procédé de préparation d'acides lactame-N-acétiques et de leurs amides répondant à la formule générale

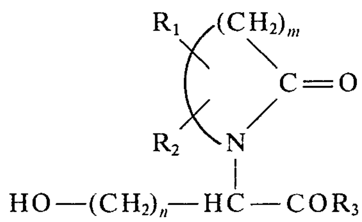

(I)

dans laquelle

$R_1$ et $R_2$ représentent indépendamment l'un de l'autre de l'hydrogène, un radical alkyle linéaire ou ramifié contenant 1 à 4 atomes de carbone, aryle non substitué ou aryle substitué par un atome d'halogène,

$R_3$ est un groupe hydroxyle ou un groupe $-NR_4R_5$ dans lequel $R_4$ et $R_5$ pris isolément représentent de l'hydrogène, un radical alkyle linéaire ou ramifié contenant 1 à 4 atomes de carbone, un radical cycloalkyle ou aralkyle, ou $R_4$ et $R_5$ pris ensemble avec l'atome d'azote auquel ils sont attachés représentent un radical hétérocyclique contenant au plus 7 chaînons, choisi parmi un radical alkylèneimino, oxaalkylèneimino, azaalkylèneimino et N-benzyl-azaalkylèneimino,

m est égal à 3, 4 ou 5,

n est égal à 0, 1 ou 2,

ainsi que des sels pharmaceutiquement acceptables desdits acides lactame-N-acétiques, caractérisé en ce que:

a) pour préparer les acides lactame-N-acétiques répondant à la formule générale I dans laquelle n est égal à 1 ou 2 et $R_3$ représente un groupe hydroxyle, on fait réagir, dans un solvant inerte, un dérivé de métal alcalin d'un lactame de formule

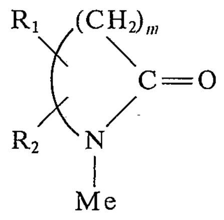

(II)

dans laquelle $R_1$, $R_2$ et m ont la signification donnée ci-dessus et Me représente un métal alcalin, avec une alpha-bromolactone de formule

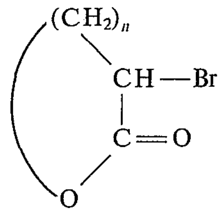

(III)

dans laquelle n a la signification donnée ci-dessus, et on soumet ensuite la lactone de l'acide alpha-(hydroxyalkyl)-lactame-N-acétique ainsi obtenue de formule

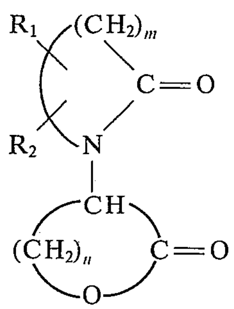

(IV)

dans laquelle $R_1$, $R_2$, m et n ont la signification donnée ci-dessus, à une hydrolyse avec un hydroxyde de métal alcalin, et on libère finalement l'acide libre par acidification du sel de métal alcalin de l'acide alpha-(hydroxyalkyl)-lactame-N-acétique ainsi obtenu de formule

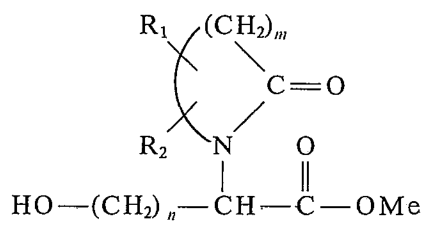

(V)

dans laquelle $R_1$, $R_2$, m et n ont la signification donnée ci-dessus et Me représente un métal alcalin; ou

b) pour préparer les amides d'acides lactame-N-acétiques répondant à la formule générale I dans laquelle n est égal à 1 ou 2 et $R_3$ représente un groupe $-NR_4R_5$, on fait réagir une lactone d'un acide alpha-(hydroxyalkyl)-lactame-N-acétique de formule IV obtenue de la manière décrite au point a) ci-dessus avec un composé azoté de formule

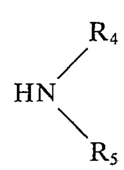

(VI)

dans laquelle $R_4$ et $R_5$ ont la signification donnée ci-dessus; ou

c) pour préparer les acides lactame-N-acétiques répondant à la formule générale I dans laquelle n est égal à 0 et $R_3$ représente un groupe hydroxyle, on fait réagir l'acide glyoxylique avec un lactame de formule

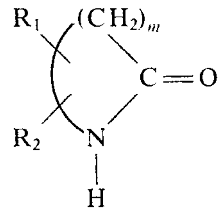

(VII)

dans laquelle $R_1$, $R_2$ et m ont la signification donnée ci-dessus; ou

d) pour préparer les amides d'acides lactame-N-acétiques répondant à la formule générale I dans laquelle n est égal à 0 et $R_3$ représente un groupe $-NR_4R_5$, on fait réagir d'abord l'acide glyoxylique ou un ester de cet acide avec un lactame de formule VII indiquée ci-dessus et on condense ensuite l'acide alpha-hydroxy-lactame-N-acétique ainsi obtenu de formule

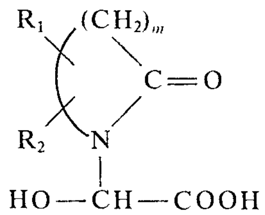

(VIII)

dans laquelle $R_1$, $R_2$ et m ont la signification donnée ci-dessus, ou l'ester correspondant, avec un composé azoté de formule VI indiquée ci-dessus; et

e) en ce que, éventuellement, on convertit les acides lactame-N-acétiques de formule I obtenus aux points a) et c) ci-dessus en leurs sels pharmaceutiquement acceptables.

**Patentansprüche für die Vertragsstaaten: BE, FR, GB, NL, DE, LU, SE, CH und IT**

1. Lactam-N-essigsäuren und ihre Amide entsprechend der allgemeinen Formel

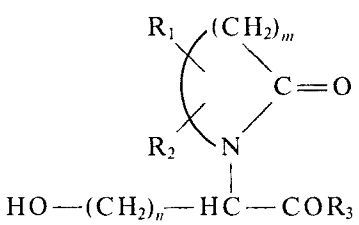

(I)

worin

$R_1$ und $R_2$ unabhängig voneinander Wasserstoff, einen geradkettigen oder verzweigten Alkylrest mit 1 bis 4 Kohlenstoffatomen, einen nichtsubstituierten Arylrest oder einen durch ein Halogenatom substituierten Arylrest darstellen,

$R_3$ ein Hydroxylrest oder der Rest $-NR_4R_5$ ist, worin $R_4$ und $R_5$ einzeln genommen Wasserstoff, einen geradkettigen oder verzweigten Alkylrest mit 1 bis 4 Kohlenstoffatomen, einen Cycloalkylrest oder einen Aralkylrest darstellen oder $R_4$ und $R_5$ zusammengenommen mit dem Stickstoffatom, an welches sie gebunden sind, einen höchstens 7gliedrigen heterocyclischen Rest, ausgewählt aus einen Alkylenimino-, Oxaalkylenimino-, Azaalkylenimino- und N-Benzyl-azaalkyleniminorest, darstellen,

m 3, 4 oder 5 ist,

n 0, 1 oder 2 ist,

sowie die pharmazeutisch annehmbaren Salze dieser Lactam-N-essigsäuren.

2. Lactam-N-essigsäuren, ihre Salze und ihre Amide nach Anspruch 1, worin m 3 ist.

3. alpha-Hydroxy-2-oxo-1-pyrrolidinessigsäure und ihre pharmazeutisch annehmbaren Salze, nach Anspruch 1.

4. alpha-(2-Hydroxyäthyl)-2-oxo-1-pyrrolidinacetamid, nach Anspruch 1.

5. N,N-Diäthyl-alpha-(2-hydroxyäthyl)-2-oxo-1-pyrrolidinacetamid, nach Anspruch 1.

6. 4-[4-Hydroxy-2-(2-oxo-1-pyrrolidinyl)butyryl]-morpholin, nach Anspruch 1.

7. N-Cyclopentyl-alpha-(2-hydroxyäthyl)-2-oxo-1-pyrrolidinacetamid, nach Anspruch 1.

8. N-Benzyl-alpha-(2-hydroxyäthyl)-2-oxo-1-pyrrolidinacetamid, nach Anspruch 1.

9. Verfahren zur Herstellung von den im Anspruch 1 bestimmten Lactam-N-essigsäuren, ihren Salzen und ihren Amiden, dadurch gekennzeichnet, daß man:

a) um Lactam-N-essigsäuren der allgemeinen Formel I herzustellen, worin n 1 oder 2 ist und $R_3$ einen Hydroxylrest darstellt, in einem inerten Lösungsmittel ein Alkalimetallderivat eines Lactams der Formel

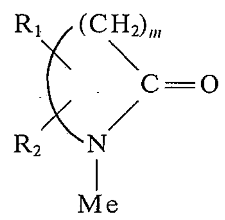

(II)

worin $R_1$, $R_2$ und m die in Anspruch 1 angegebene Bedeutung haben und Me ein Alkalimetall darstellt, mit einem alpha-Bromlacton der Formel

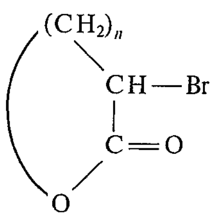

(III)

worin n die oben angegebene Bedeutung hat, reagieren läßt und anschließend das auf diese Weise erhaltene Lacton der alpha-(Hydroxyalkyl)-lactam-N-essigsäure der Formel

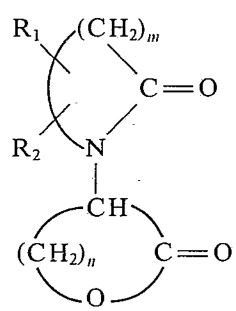

(IV)

worin $R_1$, $R_2$, m und n die oben angegebene Bedeutung haben, einer Hydrolyse mit einem Alkalimetallhydroxid unterwirft, und abschließend die freie Säure durch Ansäuern des Alkalimetallsalzes der so erhaltenen alpha-(Hydroxyalkyl)-lactam-N-essigsäure der Formel

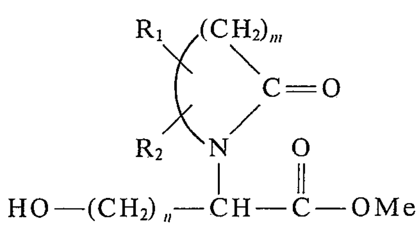

(V)

worin $R_1$, $R_2$, m und n die oben angegebene Bedeutung haben und Me ein Alkalimetall darstellt, freisetzt; oder

b) um Amide von Lactam-N-essigsäuren der allgemeinen Formel I herzustellen, worin n 1 oder 2 ist und $R_3$ einen Rest $-NR_4R_5$ darstellt, ein auf im obigen Punkt a) beschriebener Weise erhaltenes

Lacton einer alpha-(Hydroxyalkyl)-lactam-N-essigsäure der Formel IV mit einer Stickstoffverbindung der Formel

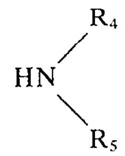

(VI)

worin $R_4$ und $R_5$ die in Anspruch 1 angegebene Bedeutung haben, reagieren läßt; oder

c) um Lactam-N-essigsäuren der allgemeinen Formel I herzustellen, worin n 0 ist und $R_3$ einen Hydroxylrest darstellt, Glyoxylsäure mit einem Lactam der Formel

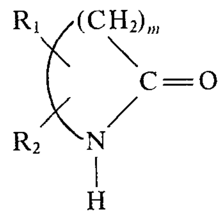

(VII)

worin $R_1$, $R_2$ und m die in Anspruch 1 angegebene Bedeutung haben, reagieren läßt; oder

d) um Amide von Lactam-N-essigsäuren der allgemeinen Formel I herzustellen, worin n 0 ist und $R_3$ einen Rest $-NR_4R_5$ darstellt, zunächst Glyoxylsäure oder einen Ester dieser Säure mit einem Lactam der oben angegebenen Formel VII reagieren läßt und anschließend die auf diese Weise erhaltene alpha-Hydroxy-lactam-N-essigsäure der Formel

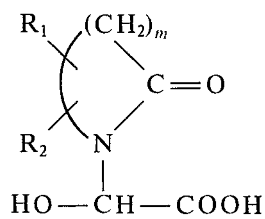

(VIII)

worin $R_1$, $R_2$ und m die oben angegebene Bedeutung haben, oder den entsprechenden Ester mit einer Stickstoffverbindung der oben angegebenen Formel VI kondensiert; und

e) gegebenenfalls die in den obigen Punkten a) und c) erhaltenen Lactam-N-essigsäuren der Formel I in ihre pharmazeutisch annehmbaren Salze umwandelt.

10. Heilmittel, enthaltend eine therapeutisch wirksame Menge einer Lactam-N-essigsäure oder ihres Amids oder eines pharmazeutisch annehmbaren Salzes dieser Lactam-N-essigsäure nach Anspruch I in Verbindung mit festen oder flüssigen, pharmazeutischen Hilfsstoffen.

**Patentanspruch für den Vertragsstaat: AT**

Verfahren zur Herstellung von Lactam-N-essigsäuren und ihren Amiden entsprechend der allgemeinen Formel

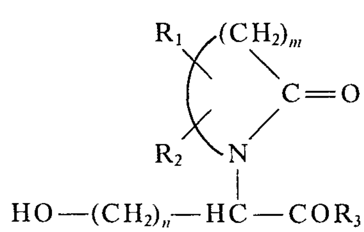

(I)

worin

$R_1$ und $R_2$ unabhängig voneinander Wasserstoff, einen geradkettigen oder verzweigten Alkylrest mit 1 bis 4 Kohlenstoffatomen, einen nichtsubstituierten Arylrest oder einen durch ein Halogenatom substituierten Arylrest darstellen,

$R_3$ ein Hydroxylrest oder der Rest $-NR_4R_5$ ist, worin $R_4$ und $R_5$ einzeln genommen Wasserstoff, einen geradkettigen oder verzweigten Alkylrest mit 1 bis 4 Kohlenstoffatomen, einen Cycloalkylrest

oder einen Aralkylrest darstellen oder $R_4$ und $R_5$ zusammengenommen mit dem Stickstoffatom, an welches sie gebunden sind, einen höchstens 7gliedrigen heterocyclischen Rest, ausgewählt aus einem Alkylenimino-, Oxaalkylenimino-, Azaalkylenimino- und N-Benzyl-azaalkyleniminorest, darstellen,

m 3, 4 oder 5 ist,

n 0, 1 oder 2 ist,

sowie die pharmazeutisch annehmbaren Salze dieser Lactam-N-essigsäuren, dadurch gekennzeichnet, daß man:

a) um Lactam-N-essigsäuren der allgemeinen Formel I herzustellen, worin n 1 oder 2 ist und $R_3$ einen Hydroxylrest darstellt, in einem inerten Lösungsmittel ein Alkalimetallderivat eines Lactams der Formel

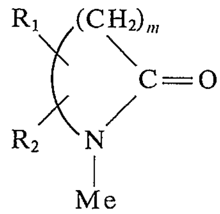

(II)

worin $R_1$, $R_2$ und m die oben angegebene Bedeutung haben und Me ein Alkalimetall darstellt, mit einem alpha-Bromlacton der Formel

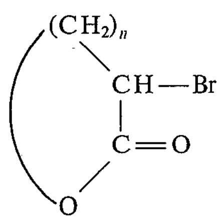

(III)

worin n die oben angegebene Bedeutung hat, reagieren läßt und anschließend das auf diese Weise erhaltene Lacton der alpha-(Hydroxyalkyl)-lactam-N-essigsäure der Formel

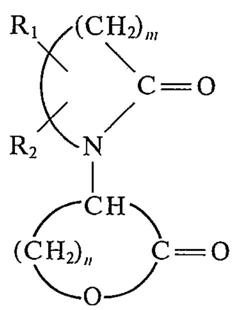

(IV)

worin $R_1$, $R_2$, m und n die oben angegebene Bedeutung haben, einer Hydrolyse mit einem Alkalimetallhydroxid unterwirft, und abschließend die freie Säure durch Ansäuern des Alkalimetallsalzes der so erhaltenen alpha-(Hydroxyalkyl)-lactam-N-essigsäure der Formel

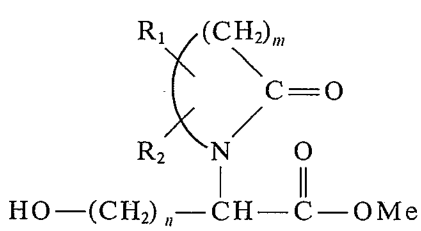

(V)

worin $R_1$, $R_2$, m und n die oben angegebene Bedeutung haben und Me ein Alkalimetall darstellt, freisetzt; oder

b) um Amide von Lactam-N-essigsäuren der allgemeinen Formel I herzustellen, worin n 1 oder 2 ist und $R_3$ einen Rest $-NR_4R_5$ darstellt, ein auf im obigen Punkt a) beschriebener Weise erhaltenes

Lacton einer alpha-(Hydroxyalkyl)-lactam-N-essigsäure der Formel IV mit einer Stickstoffverbindung der Formel

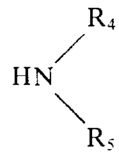

(VI)

worin $R_4$ und $R_5$ die oben angegebene Bedeutung haben, reagieren läßt; oder

c) um Lactam-N-essigsäuren der allgemeinen Formel I herzustellen, worin n 0 ist und $R_3$ einen Hydroxylrest darstellt, Glyoxylsäure mit einem Lactam der Formel

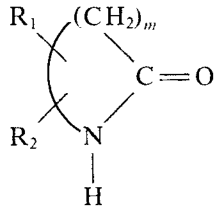

(VII)

worin $R_1$, $R_2$ und m die oben angegebene Bedeutung haben, reagieren läßt; oder

d) um Amide von Lactam-N-essigsäuren der allgemeinen Formel I herzustellen, worin n 0 ist und $R_3$ einen Rest $-NR_4R_5$ darstellt, zunächst Glyoxylsäure oder einen Ester dieser Säure mit einem Lactam der oben angegebenen Formel VII reagieren läßt und anschließend die auf diese Weise erhaltene alpha-Hydroxy-lactam-N-essigsäure der Formel

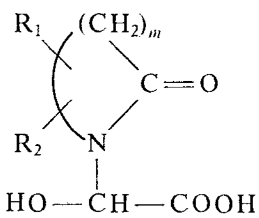

(VIII)

worin $R_1$, $R_2$ und m die oben angegebene Bedeutung haben, oder den entsprechenden Ester mit einer Stickstoffverbindung der oben angegebenen Formel VI kondensiert; und

e) gegebenenfalls die in den obigen Punkten a) und c) erhaltenen Lactam-N-essigsäuren der Formel I in ihre pharmazeutisch annehmbaren Salze umwandelt.

**Claims for the Contracting States: BE, FR, GB, NL, DE, LU, SE, CH and IT**

1. Lactam-N-acetic acids and their amides having the general formula

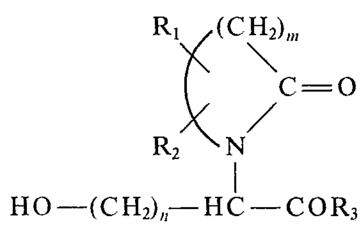

(I)

wherein

$R_1$ and $R_2$ represent independently of each other hydrogen, a straight or branched chain alkyl radical containing 1 to 4 carbon atoms, an unsubstituted aryl radical or an aryl radical being substituted by a halogen atom,

$R_3$ is a hydroxyl group or an $-NR_4R_5$ group, wherein $R_4$ and $R_5$ taken separately represent hydrogen, a straight or branched chain alkyl radical containing 1 to 4 carbon atoms, a cycloalkyl or an aralkyl radical, or $R_4$ and $R_5$, taken together with the nitrogen atom to which they are attached, represent a heterocyclic radical containing at most 7 ring members, selected from an alkyleneimino, an oxa-alkyleneimino, an azaalkyleneimino and an N-benzyl-aza-alkyleneimino radical,

m is 3, 4 or 5,

n is 0, 1 or 2,

as well as the pharmaceutically-acceptable salts of the said lactam-N-acetic acids.

2. Lactam-N-acetic acids, their salts and their amides according to claim 1, wherein m is 3.

3. alpha-Hydroxy-2-oxo-1-pyrrolidineacetic acid and its pharmaceutically-acceptable salts, according to claim 1.

4. alpha-(2-Hydroxyethyl)-2-oxo-1-pyrrolidineacetamide, according to claim 1.

5. N,N-Diethyl-alpha-(2-hydroxyethyl)-2-oxo-1-pyrrolidineacetamide, according to claim 1.

6. 4-[4-Hydroxy-2-(2-oxo-1-pyrrolidinyl)butyryl]-morpholine, according to claim 1.

7. N-Cyclopentyl-alpha-(2-hydroxyethyl)-2-oxo-1-pyrrolidineacetamide, according to claim 1.

8. N-Benzyl-alpha-(2-hydroxyethyl)-2-oxo-1-pyrrolidineacetamide, according to claim 1.

9. Process for the preparation of lactam-N-acetic acids, their salts and their amides as defined in claim 1, characterised in that:

a) in order to prepare the lactam-N-acetic acids having the general formula I, wherein n is 1 or 2 and $R_3$ represents a hydroxyl group, an alkali metal derivative of a lactam of the formula

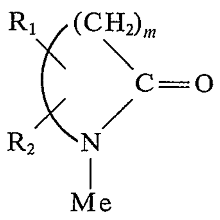

(II)

wherein $R_1$, $R_2$ and m have the meaning given in claim 1 and Me represents an alkali metal, is reacted, in an inert solvent, with an alpha-bromolactone of the formula

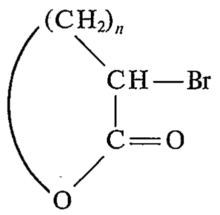

(III)

wherein n has the meaning given above, and thereafter the lactone of the alpha-(hydroxyalkyl)-lactam-N-acetic acid thus obtained of the formula

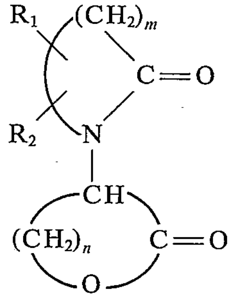

(IV)

wherein $R_1$, $R_2$, m and n have the meaning given above, is subjected to hydrolysis by means of an alkali metal hydroxide, and finally the free acid is liberated by acidification of the alkali metal salt of the alpha-(hydroxyalkyl)-lactam-N-acetic acid thus obtained of the formula

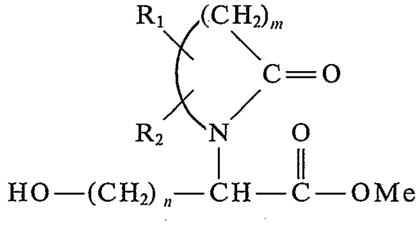

(V)

wherein $R_1$, $R_2$, m and n have the meaning given above and Me represents an alkali metal; or

b) in order to prepare the amides of lactam-N-acetic acids having the general formula I, wherein n is 1 or 2 and $R_3$ represents an $-NR_4R_5$ group, a lactone of an alpha-(hydroxyalkyl)-lactam-N-acetic acid of formula IV, obtained as described in point a) above, is reacted with a nitrogen compound of the formula

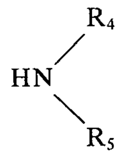

(VI)

wherein $R_4$ and $R_5$ have the meaning given in claim 1; or

c) in order to prepare the lactam-N-acetic acids having the general formula I, wherein n is 0 and $R_3$ represents a hydroxyl group, glyoxylic acid is reacted with a lactam of the formula

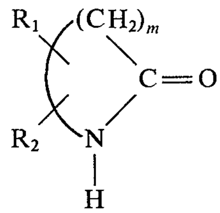

(VII)

wherein $R_1$, $R_2$ and m have the meaning given in claim 1; or

d) in order to prepare the amides of lactam-N-acetic acids having the general formula I, wherein n is 0 and $R_3$ represents an $-NR_4R_5$ group, glyoxylic acid or an ester thereof is first reacted with a lactam of formula VII given above and then the alpha-hydroxy-lactam-N-acetic acid thus obtained of formula

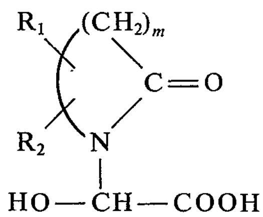

(VIII)

wherein $R_1$, $R_2$ and m have the meaning given above, or the corresponding ester, is condensed with a nitrogen compound of formula VI given above; and

e) optionally, the lactam-N-acetic acids of formula I obtained in points a) and c) above are converted into their pharmaceutically-acceptable salts.

10. Therapeutic compositions containing a therapeutically effective amount of a lactam-N-acetic acid or its amide or a pharmaceutically-acceptable salt of the said lactam-N-acetic acid according to claim 1 in association with solid or liquid pharmaceutical excipients.

**Claim for the Contracting State: AT**

Process for the preparation of lactam-N-acetic acids and their amides having the general formula

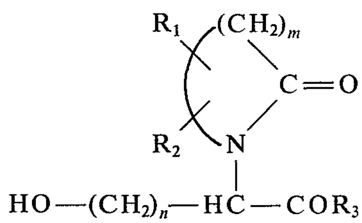

(I)

wherein

$R_1$ and $R_2$ represent independently of each other hydrogen, a straight or branched chain alkyl radical containing 1 to 4 carbon atoms, an unsubstituted aryl radical or an aryl radical being substituted by a halogen atom,

$R_3$ is a hydroxyl group or an $-NR_4R_5$ group, wherein $R_4$ and $R_5$ taken separately represent hydrogen, a straight or branched chain alkyl radical containing 1 to 4 carbon atoms, a cycloalkyl or an aralkyl radical, or $R_4$ and $R_5$, taken together with the nitrogen atom to which they are attached, represent a heterocyclic radical containing at most 7 ring members, selected from an alkyleneimino, an oxa-alkyleneimino, an azaalkyleneimino and an N-benzyl-aza-alkyleneimino radical,

m is 3, 4 or 5,

n is 0, 1 or 2,

as well as the pharmaceutically-acceptable salts of the said lactam-N-acetic acids, characterised in that:

a) in order to prepare the lactam-N-acetic acids having the general formula I, wherein n is 1 or 2 and $R_3$ represents a hydroxyl group, an alkali metal derivative of a lactam of the formula

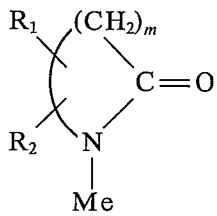

(II)

wherein $R_1$, $R_2$ and m have the meaning given above and Me represents an alkali metal, is reacted, in an inert solvent, with an alpha-bromolactone of the formula

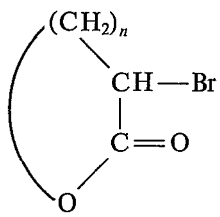

(III)

wherein n has the meaning given above, and thereafter the lactone of the alpha-(hydroxyalkyl)-lactam-N-acetic acid thus obtained of the formula

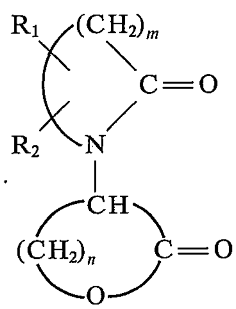

(IV)

wherein $R_1$, $R_2$, m and n have the meaning given above, is subjected to hydrolysis by means of an alkali metal hydroxide, and finally the free acid is liberated by acidification of the alkali metal salt of the alpha-(hydroxyalkyl)-lactam-N-acetic acid thus obtained of the formula

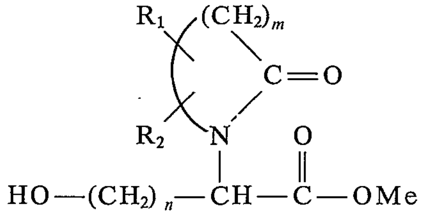

(V)

wherein $R_1$, $R_2$, m and n have the meaning given above and Me represents an alkali metal; or

b) in order to prepare the amides of lactam-N-acetic acids having the general formula I, wherein n is 1 or 2 and $R_3$ represents an $-NR_4R_5$ group, a lactone of an alpha-(hydroxyalkyl)-lactam-N-acetic acid of formula IV, obtained as described in point a) above, is reacted with a nitrogen compound of the formula

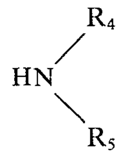

(VI)

wherein $R_4$ and $R_5$ have the meaning given above; or

c) in order to prepare the lactam-N-acetic acids having the general formula I, wherein n is 0 and $R_3$ represents a hydroxyl group, glyoxylic acid is reacted with a lactam of the formula

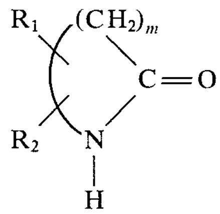

(VII)

wherein $R_1$, $R_2$ and m have the meaning given above; or

d) in order to prepare the amides of lactam-N-acetic acids having the general formula I, wherein n is 0 and $R_3$ represents an $-NR_4R_5$ group, glyoxylic acid or an ester thereof is first reacted with a lactam of formula VII given above and then the alpha-hydroxy-lactam-N-acetic acid thus obtained of formula

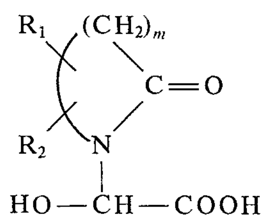

(VIII)

wherein $R_1$, $R_2$ and m have the meaning given above, or the corresponding ester, is condensed with a nitrogen compound of formula VI given above; and

e) optionally, the lactam-N-acetic acids of formula I obtained in points a) and c) above are converted into their pharmaceuticallyacceptable salts.